# EUROPEAN PATENT APPLICATION

(11) **EP 0 779 069 A1**
(43) Date of publication of application: **18.06.1997**
(21) Application number: 95930702.6
(22) Date of filing: 05.09.1995
(51) Int. Cl.: A61K 7/06

(54) **HAIR MOTHER CELL ACTIVATOR AND METHOD OF ACTIVATING HAIR MOTHER CELL**

(30) Priority: 06.09.1994 JP 212515/94
(71) Applicant: KUREHA KAGAKU KOGYO KABUSHIKI KAISHA, Chuo-ku Tokyo 103 (JP)
(72) Inventor: WATANABE, Takeo, Iwaki-shi Fukushima-ken 974 (JP); YONENO, Tadashi, Fukushima-ken 974 (JP); HATANAKA, Mituo, Fukushima-ken 974 (JP); TAKAHASHI, Eisaku, Tokio 176 (JP); KIMURA, Fumihiko, Shinjuku-ku Tokio 169 (JP); CHIBA, Tadahiko, Yono-shi Saitama-ken 338 (JP)
(74) Representative: Baverstock, Michael George Douglas
(86) International application number: JP9501765
(87) International publication number: WO9607393

(57) **Abstract**

An agent for activating hair matrix cell, comprising: a formulation A which comprises, at least a blood circulation promoter, and a cell activator, and is to be applied to hair matrix cell; and a formulation B comprising, as main effective components, a water-soluble polymer, and at least one component selected from the group consisting of monosaccharides, disaccharides and oligosaccharides; the formulation B being to be applied to hair matrix cell with a time interval between the applications of the formulations A and B. A plurality of different types of formulations A and B are combined, and these different types of formulations A and B are applied to hair matrix cell with a time interval between the applications of these formulations, thereby to enable the activation of hair matrix cell based on the a function (resting phase-breaking function) of converting the resting phase into growth phase in the hair cycle (hair growth promoting or hair-growing).

## Description

### Technical Field

The present invention relates to an agent for activating hair matrix (or mother) cells, and a method of activating hair matrix cells. The present invention is applicable to in vivo or in vitro activation of hair matrix cells (promotion of cell division of hair matrix cells) in animals such as human being (including various kinds of pet animals, mink, etc.).

### Background Art

With respect to the in-vivo activation of hair matrix cells (or production/growth of hair), there have heretofore been proposed many species of hair growth promoting agents or hair-nourishing agents (most of which are compositions), as disclosed in, e.g., Japanese Patent Publication (JP-B) Nos. Hei 6-4521 (4521/1994) to 6-4524 (4524/1994), Japanese Patent Publication Nos. Hei 6-4526 (4526/1994) to 6-4528 (4528/1994), etc.

It has been observed that some of these conventional hair growth promoting or hair-nourishing agents show a certain effect of preventing alopecia (or baldness) or promoting hair growth, but the degree or magnitude of such an effect is not necessarily satisfactory. The reason for such insufficiency is considered to be that the cause of male-type alopecia includes complicated entanglement of various kinds of factors such as imbalance between sex hormones, incompetence of blood circulation, decrease in the activity of hair matrix cells, etc. Accordingly, at present, there has been desired a more effective hair-growing or hair-producing (or hair-developing) prescription.

Further, very few examples of the conventional hair growth promoting or hair-nourishing agents have shown a function (resting phase-breaking function) of converting hair in the resting phase (or resting period, telogen) into that in the growth phase (or growth period, anagen) in its hair cycle. In other words, very few examples of these agents have shown an effect of producing hair (or developing hair).

An object of the present invention is to provide an agent for activating hair matrix cells and a method of activating hair matrix cells, which has solved the above-mentioned problems encountered in the prior art.

Another object of the present invention is to provide an agent for activating hair matrix cells or a method of activating hair matrix cells, which is capable of showing an effect of producing hair on the basis of a function (resting phase-breaking function) of converting hair in the resting phase into that in the growth phase in its hair cycle.

### Disclosure of Invention

As a result of earnest study, the present inventors have found that it is extremely effective in achieving the above objects not only to combine a plurality of different types of formulations, i.e., a formulation A having a composition suitable for a tonic preparation (a preparation comprising an alcohol as a liquid component), and another formulation B having a composition suitable for a lotion preparation (a preparation comprising an aqueous solvent as a liquid component); but also to apply these different formulations of A and B to hair matrix cells with an interval of time between the applications of these formulations; i.e., to repeatedly provide different stimuli to hair matrix cells.

The hair matrix cell activating agent according to the present invention is based on the above discovery, and comprises:
a formulation A which comprises, at least a blood circulation promoter, and a cell activator, and is to be applied to hair matrix cell; and
a formulation B comprising, as main effective components, a water-soluble polymer, and at least one component selected from the group consisting of monosaccharides, disaccharides and oligosaccharides; the formulation B being to be applied to hair matrix cell with a time interval between the applications of the formulations A and B.

The present invention also provides a method of activating hair matrix cell, wherein a formulation A comprising at least, a blood circulation promoter is applied to hair matrix cell; and a formulation B comprising, as main effective components, a water-soluble polymer, and at least one component selected from the group consisting of monosaccharides, disaccharides and oligosaccharides is applied to hair matrix cell after 1 to 36 hours counted from the application of the formulation A.

The present invention further provides a method of activating hair matrix cell, wherein a formulation B comprising, as main effective components, a water-soluble polymer, and at least one component selected from the group consisting of monosaccharides, disaccharides and oligosaccharides is applied to hair matrix cell; and a formulation A comprising at least, a blood circulation promoter is applied to hair matrix cell after 1 to 36 hours counted from the application of the formulation B.

In the present invention, the term "to apply to hair matrix cell" refers to that the above-mentioned formulation A or B is brought into physical contact with hair matrix cell in vivo or in vitro. The means for achieving this physical contact is not particularly limited. In other words, it is possible to employ known means such as direct application, sprinkling or spraying; physical contact based on penetration or diffusion through another portion such as skin (epidermis, dermis), and subcutaneous tissue.

In the present invention, the activation of hair matrix cell refers to the promotion or acceleration of cell division of hair matrix cells [or hair production (or hair development) or hair growth based on the promotion of the cell division]. This activation may include either of: the direct effect of the application of the formulation A or B to hair matrix cells; indirect effect of the promotion of blood circulation based on the vasodilation or widening of blood capillary adjacent to the hair matrix cells (e.g., blood capillary distributed in hair papilla); or a combination of these effects.

### Brief Description of Drawings

Fig. 1 (Table 1) is a table showing the compositions of tonic preparations prepared in Examples appearing hereinafter.

Fig. 2 (Table 2) is a table showing the compositions of lotion preparations prepared in Examples appearing hereinafter.

Fig. 3 (Table 3) is a table showing the results of hair growth promoting test wherein the above-mentioned tonic or lotion preparation was applied onto skins of backs of mice.

Fig. 4 (Table 4) is a table showing the results of hair growth promoting test wherein the above-mentioned tonic or lotion preparation was applied onto scalps of human beings.

### Best Mode for Carrying Out the Invention

Hereinbelow, the present invention will be described in detail with reference to the accompanying drawings as desired. In the following description, % and part(s) representing a quantitative proportion or ratio are those based on mass (i.e., % by mass or part(s) by mass), unless otherwise noted specifically. (Hair matrix cell-activating agent)

As described above, the hair matrix cell-activating agent according to the present invention comprises a formulation A having a composition suitable for a tonic preparation, and a formulation B which has a composition suitable for a lotion preparation and is one to be applied to hair matrix cells with an interval of time between the application thereof and the application of the above formulation A.

In view of the efficiency in the promotion of hair production or hair growth, it is preferred that these formulations A and B are alternately applied to hair matrix cells. In the order or sequence of the applications of these formulations to hair matrix cells, either of the applications of the formulations of A and B can be the preceding application. In other words, the order may be either of A → B → A → ·····, or B → A → B → ·····.

The length of the interval between the time point of the application of the formulation A to hair matrix cells, and the time point of the application of the formulation B to hair matrix cells may preferably be about 1 to 36 hours (more preferably, about 2 to 20 hours).

### (Formulation A)

The formulation A having a composition suitable for a hair tonic preparation comprises: at least, a blood circulation-promoter (or stimulator), and a cell activator. The formulation A has mainly a function of promoting hair growth. More specifically, the formulation A has mainly a function of promoting the metabolism of a hair root which is in the growth phase in its hair cycle so as to prevent epilation or depilation, and to promote hair growth. The formulation A may more preferably comprise another component such as skin stimulant, humectant or humectant, and anti-inflammatory agent.

### (Blood circulation promoter)

The blood circulation promoter is a component for promoting the blood circulation toward the hair papilla which has mainly a function of providing a nutrient to the hair matrix cell (or for vasodilating (or widening) blood capillary in the hair papilla. The blood circulation promoter may be used in a combination of two or more species thereof, as desired.

As the above blood circulation promoter, it is possible to use at least one species appropriately selected from known blood circulation promoters including: compounds or compositions such as swertia herb extract (or swertinogen), hinokitiol, vitamin E derivative (in the present specification, this term is used so as to also include "vitamin E" per se. For example, vitamin E acetate, vitamin E nicotinate), garlic extract, cepharanthin, carpronium chloride, minoxizil, Ligusticum acutilobum root extract, ginseng extract, gentian extract, and acetylcholine,

In the present invention, in view of the synergistic effect with another component constituting the formulation A, it is preferred to select at least one species of from swertia herb extract, hinokitiol, and vitamin E derivative, among the above-mentioned components.

The above-mentioned blood circulation promoter may preferably be used in an amount of about 0.1 - 4 % (more preferably, about 0.25 - 1.0 %) based on the entire mass of the formulation A (in a case where two or more components are used as the blood circulation promoter, this amount refers to the total amount of these components; this is the same with respect to the amounts of the other components constituting the formulations A and B appearing hereinbelow).

### (Skin stimulant)

The skin (or local) stimulant is a component having a function of stimulating a site to which the formulation A has been applied. The skin (local) stimulant may be used in a combination of two or more species thereof, as desired.

As the above skin (local) stimulant, it is possible to use at least one species appropriately selected from known skin stimulants including: compounds or compositions such as L (el)- menthol, capsicum tincture, peppermint (or mentha) oil, cantharis tincture, benzyl nicotinate, ginger tincture, nonylic acid vanillylamide, and camphor. In the present invention, in view of the synergistic effect with another component constituting the formulation A, it is preferred to use at least one species selected from L-menthol, capsicum tincture and peppermint oil, among the above-mentioned components.

It is preferred to use the above-mentioned skin stimulant in an amount of about 0.01 - 4 % (more preferably, about 0.025 - 1.0 %) based on the entire mass of the formulation A.

### (Cell activator)

The cell activator is a component having a function of activating the hair matrix cell. The cell activator may be used in a combination of two or more species thereof, as desired.

As the above cell activator, it is possible to use at least one species appropriately selected from known cell activators including: compounds and compositions such as D-pantothenyl alcohol derivative (in the present specification, this term is used so as to also include "D-panthothenyl alcohol" per se), placenta extract, photosensitizing dye No. 301, biotin, pentadecanoic acid glyceride, and ginseng extract. In the present invention, in view of the synergistic effect with another component constituting the formulation A, it is preferred to use at least one species selected from D-panthothenyl alcohol derivative (D-panthothenyl alcohol and/or D-panthothenyl ethyl ether), and placenta extract, among the above-mentioned components.

It is preferred to use the above-mentioned cell activator in an amount of about 0.05 - 5 % (more preferably, about 0.1 - 1 %) based on the entire mass of the formulation A.

### (Humectant)

The humectant is a water-soluble substance having a high absorptivity, which is to be used for the purpose of providing water content to skin or hair so as to prevent dryness thereof. The humectant may be used in a combination of two or more species thereof, as desired.

As the above humectant, it is possible to use at least one species appropriately selected from known humectants including: compounds and compositions such as glycerin, propylene glycol, hyaluronic acid (this term is used so as to also include hyaluronic acid salt), pyrrolidone carboxylic acid sodium salt, and Mini-Sasanishiki extract. In the present invention, in view of the synergistic effect with another component constituting the formulation A, it is preferred to use at least one species selected from glycerin, propylene glycol, and hyaluronic acid, among the above-mentioned components.

The above-mentioned humectant may preferably be used in an amount of about 0.5 - 5 % (more preferably, about 1 - 3 %) based on the entire mass of the formulation A.

### (Anti-inflammatory agent)

The anti-inflammatory agent (antiphlogistic agent) is a component having an anti-inflammatory activity. The anti-inflammatory agent may be used in a combination of two or more species thereof, as desired.

As the above anti-inflammatory agent, it is possible to use at least one species appropriately selected from known anti-inflammatory agents including: compounds and compositions such as glycyrrhizic acid derivative (This term is used so as to also include "glycyrrhizic acid" per se), glycyrrhiza (or licorice) extract, carbenoxolone di-sodium, guaiazulene, diphenhydramine hydrochloride, lithospermum root extract, and rosa fruit extract. In the present invention, in view of the synergistic effect with another component constituting the formulation A, it is preferred to use at least one species selected from glycyrrhizic acid derivative (particularly, glycyrrhizic acid di-potassium) and licorice extract, among the above-mentioned components.

The above-mentioned anti-inflammatory agent may preferably be used in an amount of about 0.05 - 3 % (more preferably, about 0.1 - 2 %) based on the entire mass of the formulation A.

### (Preferred combinations of formulation A components)

In the present invention, it is particularly preferred to use the combination of the following components.
〈Blood circulation promoter〉 : swertia herb extract, hinokitiol, vitamin-E niconinate, vitamin-E acetate;
〈Skin stimulant〉: peppermint oil, L(el)-menthol, benzyl nicotinate;
〈Cell activator〉: D-panthothenyl alcohol, D-panthothenyl ethyl ether, ginseng extract;
〈Humectant〉: glycerin + propylene glycol
〈Anti-inflammatory agent〉: glycyrrhizic acid di-potassium, glycyrrhizic acid.

### (Other component)

In view of further enhancement of the effect in combination with the formulation B, it is more preferred to incorporate into the formulation A at least one species of component (such as trehalose) selected from "monosaccharides, disaccharides and oligosaccharides" as described hereinafter; and/or a water-soluble polymer (such as polyvinylpyrroridone). In such an embodiment, it is preferred to use about 0.5 - 10 % (more preferably, about 1.0 - 5 %) of the "monosaccharide, disaccharide or oligosaccharide"; and about 0.25 - 5 % (more preferably, about 0.5 - 2.5 %) of the water-soluble polymer, based on the entire mass of the formulation A. Further, it is also possible to incorporate into the formulation A another component such as disinfectant (or germicide) and nutrient.

### (Formulation B)

The formulation B having a composition suitable for a lotion preparation comprises, as main effective components, at least one species selected from "monosaccharides, disaccharides and oligosaccharides"; and at least one species selected from water-soluble polymers. The formulation B has mainly a function of breaking the resting phase. More specifically, the formulation B has mainly a function of promoting the metabolism of a hair root which is in the resting phase in its hair cycle, and of converting the hair cycle thereof into the growth phase.

### (Monosaccharide, disaccharide, and oligosaccharide)

As the above-mentioned "oligosaccharide", it is possible to use a polysaccharide which has been produced by the condensation of 9 (nine) or less monosaccharide molecules. In the present invention, as the above monosaccharide, disaccharide or oligosaccharide", it is possible to use at least one species appropriately selected from known monosaccharides, disaccharides, and oligosaccharides, which include: monosaccharides such as glucose, xylose, fructose, galactose, mannose, and arabinose; disaccharides such as sucrose, lactose, palatinose, maltose, cellobiose, and trehalose; trisaccharides such as raffinose; oligosaccharides such as fructo-oligosaccharides, isomalto-oligosaccharides, xylo-oligosaccharides, dextrins, cyclodextrins (α-, β-, or γ-cyclodextrin; glucose unit = 6 to 9); etc. These saccharides may be used alone or in a mixture of two or more species thereof. In a case where each of these saccharides contains isomers, such an isomer may be used alone or in a mixture with another isomer(s). As these saccharides, it is possible to use one which has been prepared by using a known method (method described in literature), and it is also possible to use a commercially available saccharide (after the purification thereof, as desired).

In the present invention, in view of the synergistic effect with another component constituting the formulation B, it is preferred to use at least one species selected from trehalose and cyclodextrins, among the above-mentioned components.

It is preferred to use the above "monosaccharide, disaccharide or oligosaccharide" in an amount of about 0.5 - 20 %, (more preferably, about 1.0 - 10 %) based on the entire weight of the formulation B.

### (Water-soluble polymer)

As the above-mentioned water-soluble polymer, it is preferred to use a polysaccharide (which refers to a polysaccharide which has been obtained by the condensation of 10 (ten) or more monosaccharide molecules) and/or a synthetic polymer. The water-soluble polymer may also be used in a combination of two or more species thereof, as desired.

As the above-mentioned polysaccharide, it is preferred to use at least one species selected from natural products or derivatives thereof such as: methyl cellulose, CMC (carboxymethyl cellulose) derivatives, hydroxypropyl cellulose, alginic acid derivative, Tamarindus indica seed polysaccharides, and chitosan. On the other hand, as the above-mentioned synthetic polymer, it is preferred to use at least one species selected from synthetic polymers and their derivatives such as poly(vinyl alcohol) and polyvinylpyrroridone.

In the present invention, in view of the synergistic effect with another component constituting the formulation B, it is preferred to use at least one species selected from alginic acid derivatives (such as sodium alginate and alginic acid propylene glycol ester), CMC derivatives (such as carboxymethyl cellulose, sodium salt), Tamarindus indica seed polysaccharide (Tamarindus indica gum), chitosan (to be used in combination with an acidic compound such as acetic acid, for the purpose of solubilization thereof), poly(vinyl alcohol), and polyvinylpyrroridone, among the above-mentioned water-soluble polymers.

It is preferred to use the above-mentioned " polysaccharide or water-soluble polymer" in an amount of about 0.1 - 10 % (more preferably, about 0.5 - 5 %) based on the entire weight of the formulation B.

### (Preferred combination of formulation B components)

In the present invention, it is particularly preferred to use the combination of following components.

### 〈monosaccharide, disaccharide, oligosaccharide〉

### 〈polysaccharide, water-soluble polymer〉

- Trehalose: Tamarindus indica gum
- Trehalose: Chitosan
- Trehalose: CMC-Na
- Trehalose: Alginic acid, sodium salt
- Trehalose: Poly(vinyl alcohol)
- Trehalose: Polyvinylpyrroridone
- Trehalose: Poly(vinyl alcohol)
- Alginic acid: Poly(propylene glycol)ester

### (Other component)

As described above, the formulation B comprises at least one species selected from the monosaccharides, disaccharides, and oligosaccharides; and at least one species selected from the polysaccharides and water-soluble polymers. The formulation B may also comprise another component, as desired. In view of further enhancement of the effect in combination with the formulation A as described hereinabove, the formulation B may more preferably comprise a blood circulation promoter (such as swertia herb extract and ginseng extract). In such an embodiment, it is preferred to use the blood circulation promoter in an amount of about 0.1 - 3 % (more preferably, about 0.25 - 2 %) based on the total weight of the formulation B.

### (Preparation form)

The above-mentioned formulation A or B can be used in an arbitrary preparation form such as aqueous solution, aqueous suspension, emulsion, cream, gel, and aerosol, but the formulation A may preferably be used in the preparation form of a tonic. On the other hand, the formulation B may preferably be used in the preparation form of a lotion. In the present invention, the tonic preparation refers to an external-use liquid formulation which comprises a solvent containing 50 (weight) % or more of alcohol, and respective components which have been dissolved or minutely uniformly dispersed in the solvent. The content of the alcohol may preferably be 60 % or more (more preferably 65 % or more) based on the total weight of the solvent. In general, the appearance of such a tonic preparation is clear or transparent.

As the alcohol constituting the above-mentioned tonic preparation, it is possible to use a known liquid component having alcoholic hydroxyl group alone or in a combination of two or more species thereof, without particular limitation. However, in view of safety or volatility, the alcohol may preferably comprise 40 % or more (more preferably, 60 % or more) of ethanol (C2H5OH).

On the other hand, the above-mentioned "lotion preparation" refers to an external-use liquid formulation which comprises an aqueous solvent, and respective components dissolved or minutely uniformly dispersed in the solvent. The appearance of such a lotion preparation may be any of solution state (this term is used so as to include "colloidal solution"), milk or latex-like liquid, suspension liquid, etc.

The water content of the above "aqueous solvent" constituting the above-mentioned lotion preparation may preferably be 90 % or more (more preferably 95 % or more) based on the total weight of the aqueous solvent.

In the present invention, it is also possible to add another known component as desired, in order to maintain the preparation form as described above.

Further, it is also possible to add a known additive such as anti-oxidant, antiseptic, colorant, stabilizer, solubilizer, viscosity adjustant, refreshing agent, and perfume, as desired.

Hereinbelow, the present invention will be described in more detail with reference to Examples. However, the present invention is not restricted by these Examples.

In the following Examples, respective commercially available products were used as the following components.
Hinokitiol: mfd. by Kanto Kagaku K.K.
Vitamin E nicotinate: mfd. by Wako Junyaku K.K. Mentha oil: mfd. by Nikko Seiyaku K.K.
Glycyrrhizic acid di-potassium: mfd. by Sanyo-Kokusaku Pulp K.K.
D-panthothenyl alcohol: mfd. by Wako Junyaku K.K.
Pyridoxine hydrochloride (vitamin B6): mfd. by Wako Junyaku K.K.
Methylparaben (preservative): mfd. by Tokyo Kasei K.K.
Tamarindus indica gum: Glyloid-3S (trade mark): mfd. by Dainippon Seiyaku K.K.
α, α-trehalose (TRH): mfd. by Sigma Co.
Hydroxypropyl cellulose (HPC): (L) type, mfd. by Nihon Soda K.K., Lot. No.HE-341
Swertia extract: Swertia Extract Liquid, mfd. by Maruzen Seiyaku K.K.
Chitosan: Chitosan-PSH (trade mark), mfd. by Yaizu Suisan Kagaku Kogyo K.K.
Alginic acid, sodium salt: mfd. by Wako Junyaku K.K.
Carboxymethyl cellulose, sodium salt (CMC-Na): Dai-ichi Kogyo Seiyaku K.K.
Poly(vinyl alcohol) (PVA): Poval-117 mfd. by Kuraray K.K.
Polyvinylpyrroridone: Polyvinylpyrroridone (K-90) mfd. by Wako Junyaku Kogyo K.K.
Alginic acid propylene glycol ester (PGA): mfd. by Kibun Food Chemical K.K., LFM

### Examples

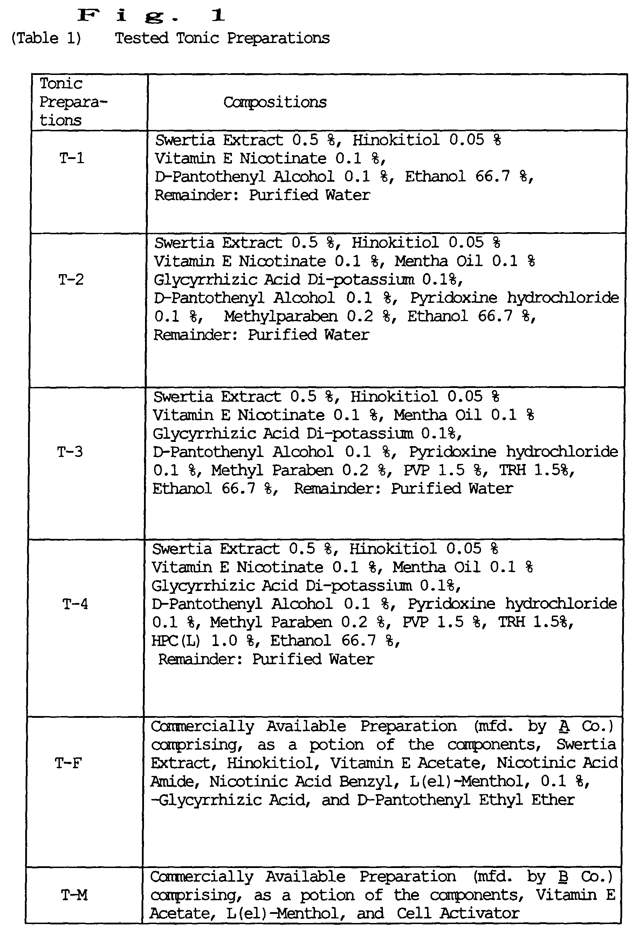

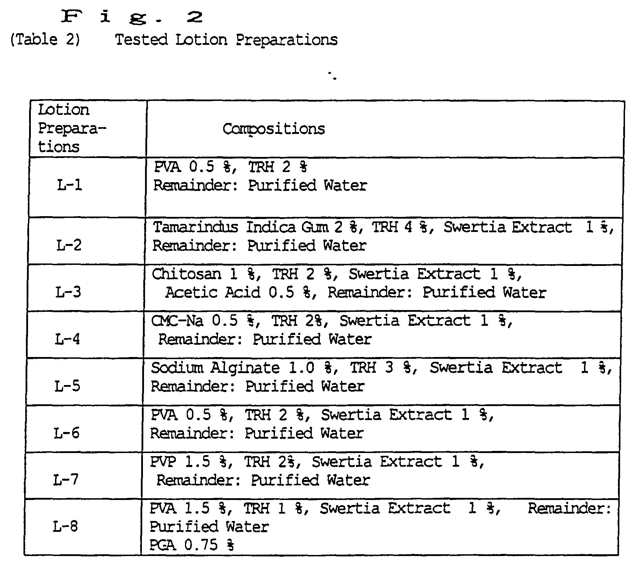

The respective components as shown in Fig. 1 (Table 1) and Fig. 2 (Table 2) were mixed with each other, thereby to prepare formulations A in the preparation form of a tonic (T-1 to T-4, T-F and T-M), and to prepare formulations B in the preparation form of a lotion (L-1 to L-8).

By use of the above-mentioned formulations A and B, hair growth promoting tests were conducted by using hair-cut back portions of C3H-type mice which were in the phase of resting phase in the hair cycle, thereby to evaluate the effect of the hair matrix cell activation (hair growth promoting effect). More specifically, these tests were conducted in the following manner.

### Hair growth promoting effect test (mice)

With respect to each of test sections (corresponding to each of combinations of formulations A and B), skins of backs of respective 10 (ten) C3H/NeH mice (male, 8-weeks old) were subjected to hair-cutting by means of an electric hair clippers (trade name: National Suki-Karu B-Cut ER552HP having ceramic edges) so as to remove the hair (dimensions: about 2.5 cm × about 5.5 cm). At this time, it was confirmed that each of the back skins was in the resting phase (i.e., each skin was colored pink). In this examination system, hair production is never observed for about 14 weeks in each hair-cut skin, when no drug was applied, or water or alcohol is applied thereto.

Onto the left half portion of each of the above-mentioned mice back skins which had been subjected to hair removal, the respective combinations of the tonic preparations shown in the above-mentioned Fig. 1 (Table 1) (T) and lotion preparations shown in Fig. 2 (Table 2) (L) were alternately applied by use of a finger-tip in accordance with each of the combinations shown in the following Table 3, with a time interval between the respective applications.

The application of each of the formulations was started from the next day of the above-mentioned hair removal. The amount of the application of each formulation used was 0.1 ml per one mouse per one application. The application was conducted in the morning (at 10:00 a.m.) with respect to the above-mentioned Table 3 "test Nos. 1-5", while the application was conducted in the evening (at 4:00 p.m.) with respect to "test Nos. 6-12" with a frequency of one application per one day. On the other hand, with respect to the "test Nos. 13-30", both of the morning application and evening application were conducted so as to provide a frequency of one T-formulation morning application per one day and L-formulation evening application per one day.

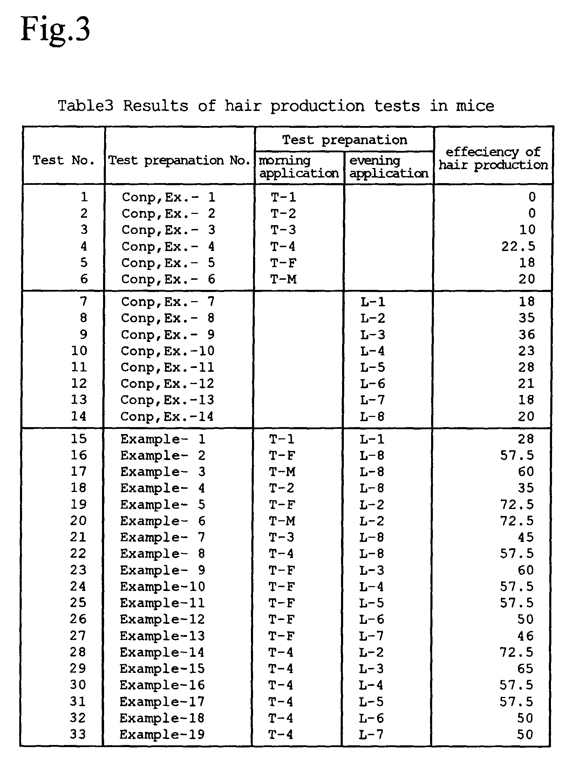

When 42 days (No. of applications: 42 or 84) elapsed after the initiation of the above application, the area showing hair production was measured, and the ratios of the hair growth promoting areas (area of hair growth promoting portion/total area of application portion) were calculated, thereby to evaluate the effect of each application of the above formulation. The thus obtained results are shown in Fig. 3 (Table 3).

The area of the above-mentioned hair growth promoting portion was measured in the following manner.

### 〈Method of measuring area of hair growth promoting portion〉

The area of each hair growth promoting portion was measured by using a frame having a side of 12.5 mm and a length of 27.5 mm with a 1.0 mm-mesh.

As shown in the above-mentioned Fig. 3 (Table 3), a remarkable hair growth promoting effect was recognized when the formulation A (tonic preparation) and formulation B (lotion preparation) were applied to hair matrix cells in vivo (mice back) with a time interval between the respective applications. Since the above-mentioned test was conducted by using mice which were in the resting phase in their hair cycle, it was considered that the hair growth promoting effect observed in the above test was based on the activation of hair matrix cells caused by a function of converting the resting phase into growth phase in the hair cycle (resting phase-breaking effect).

### Practical use test for human being

Onto each of scalps (mainly onto each of the portions from which hair had been shed) of seven examinees of male-type alopecia, "T-4" formulation shown in the above-mentioned Fig. 1 (Table 1) was applied in the morning (at 7:00 a.m.), and the "L-2" formulation shown in the above-mentioned Fig. 1 (Table 1) was applied at night (at 9:00 p.m.), respectively in an amount of 0.5 - 1.0 ml each time. The application was conducted so as to provide 5 to 6 sets per one week, wherein the above-mentioned "morning and night applications" was treated as one set. The period for the application was 5 - 9 months.

The evaluated items were four, including: (1) dandruff prevention effect, (2) epilation prevention effect, (3) hair-nourishing feeling, and (4) hair growth promoting effect. With respect to these four items, the same observer conducted the evaluation by visual observation. The criteria of the evaluation were as follows.
ⓞ: Remarkable effect was observed.
○: A certain effect was observed.
-: No conspicuous change was observed.

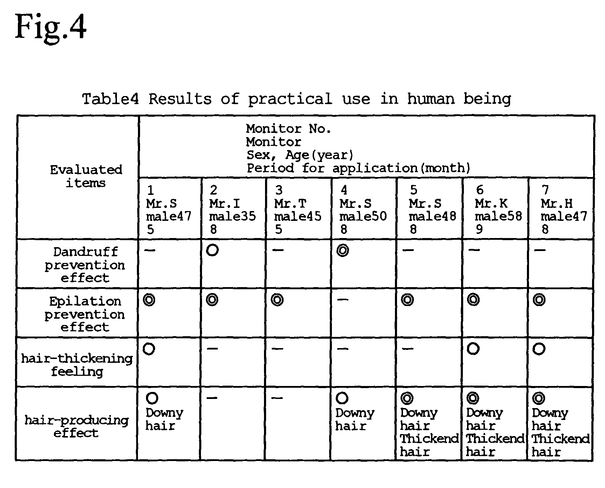

The thus obtained results are shown in Fig. 4 (Table 4).

As shown in Fig. 4 (Table 4), remarkable hair growth promoting effect or epilation prevention effect was recognized, when the formulation A (tonic preparation) and the formulation B (lotion preparation) were applied to hair matrix cells in vivo (head portion of human being).

### Industrial Applicability

As described hereinabove, according to the present invention, there is provided an agent for activating hair matrix cell, comprising: a formulation A which comprises, at least a blood circulation promoter, and a cell activator, and is to be applied to hair matrix cell; and a formulation B comprising, as main effective components, a water-soluble polymer, and at least one component selected from the group consisting of monosaccharides, disaccharides and oligosaccharides; the formulation B being to be applied to hair matrix cell with a time interval between the applications of the formulations A and B.

The present invention also provides a method of activating hair matrix cell, wherein one of the above formulations A and B is applied to hair matrix cell; and the other of the above formulations A and B is applied to hair matrix cell after 1 to 36 hours counted from the above application.

According to the present invention, a plurality of different types of formulations A and B are combined, and simultaneously these different types of formulations A and B are applied to hair matrix cells with a time interval between the applications of these formulations, thereby to enable the activation of hair matrix cells based on the a function (resting phase-breaking function) of converting the resting phase into growth phase in the hair cycle (hair growth promoting or hair-growing).

The hair matrix cell activator and hair matrix cell activating method according to the present invention are usable both in vitro and in vivo. In in-vivo application, the present invention is applicable to the activation of hair matrix cells of animals such as human being (e.g., hair production or hair growth of fur animals such as mink, epilation prevention of pet animals, etc.).

## Claims

1. An agent for activating hair matrix cell, comprising:
a formulation A which comprises, at least a blood circulation promoter, and a cell activator, and is to be applied to hair matrix cell; and
a formulation B comprising, as main effective components, a water-soluble polymer, and at least one component selected from the group consisting of monosaccharides, disaccharides and oligosaccharides; the formulation B being to be applied to hair matrix cell with a time interval between the applications of the formulations A and B.

2. An agent for activating hair matrix cell according to Claim 1, wherein the water-soluble polymer is a synthetic polymer.

3. An agent for activating hair matrix cell according to Claim 1, wherein the water-soluble polymer is a polysaccharide.

4. An agent for activating hair matrix cell according to Claim 1, wherein the formulation A has the preparation form of a tonic, and the formulation B has the preparation form of a lotion.

5. An agent for activating hair matrix cell according to Claim 1, wherein the blood circulation promoter is at least one component selected from the group consisting of: swertia herb extract, hinokitiol and vitamin E derivative;
the cell activator is at least one component selected from the group consisting of: D-panthothenyl alcohol or D-panthothenyl ethyl ether, and placenta extract;
the water-soluble polymer is at least one component selected from the group consisting of: alginic acid derivative, CMC (carboxymethyl cellulose) derivative, Tamarindus indica seed polysaccharide, chitosan, poly(vinyl alcohol), and polyvinylpyrroridone; and
the monosaccharide, disaccharide or oligosaccharide is at least one component selected from the group consisting of: trehalose and cyclodextrin.

6. A method of activating hair matrix cell, wherein the formulation A according to Claim 1 is applied to hair matrix cell; and
the formulation B according to Claim 1 is applied to hair matrix cell after 1 to 36 hours counted from the application of the formulation A.

7. A method of activating hair matrix cell, wherein the formulation B according to Claim 1 is applied to hair matrix cell; and
the formulation A according to Claim 1 is applied to hair matrix cell after 1 to 36 hours counted from the application of the formulation B.

8. A method of activating hair matrix cell according to Claim 6 or 7, wherein the water-soluble polymer is a synthetic polymer.

9. A method of activating hair matrix cell according to Claim 6 or 7, wherein the water-soluble polymer is a polysaccharide.

10. A method of activating hair matrix cell according to Claim 6 or 7, wherein the formulation A has the preparation form of a tonic, and the formulation B has the preparation form of a lotion.

11. A method of activating hair matrix cell according to Claim 6 or 7, wherein the blood circulation promoter is at least one component selected from the group consisting of: swertia herb extract, hinokitiol and vitamin E derivative;
the cell activator is at least one component selected from the group consisting of: D-panthothenyl alcohol or D-panthothenyl ethyl ether, and placenta extract;
the water-soluble polymer is at least one component selected from the group consisting of: alginic acid derivative, CMC (carboxymethyl cellulose) derivative, Tamarindus indica seed polysaccharide, chitosan, poly(vinyl alcohol), and polyvinylpyrroridone; and
the monosaccharide, disaccharide or oligosaccharide is at least one component selected from the group consisting of: trehalose and cyclodextrin.

## Amended claims

### Amended claims under Art. 19.1 PCT

1. An agent for activating hair matrix cells, comprising:
a formulation A which comprises, at least a blood circulation promoter, and a cell activator, and is to be applied to hair matrix cells; and
a formulation B comprising, as main effective components, a water-soluble polymer, and at least one component selected from the group consisting of monosaccharides, disaccharides and oligosaccharides; the formulation B being to be applied to the same hair matrix cells with a time interval between the applications of the formulations A and B.

2. An agent for activating hair matrix cells according to Claim 1, wherein the water-soluble polymer is a synthetic polymer.

3. An agent for activating hair matrix cells according to Claim 1, wherein the water-soluble polymer is a polysaccharide.

4. An agent for activating hair matrix cells according to Claim 1, wherein the formulation A has the preparation form of a tonic comprising an alcohol as a liquid component, and the formulation B has the preparation form of a lotion comprising an aqueous solvent as a liquid component.

5. An agent for activating hair matrix cells according to Claim 1, wherein the blood circulation promoter is at least one component selected from the group consisting of: swertia herb extract, hinokitiol and vitamin E derivative;
the cell activator is at least one component selected from the group consisting of: D-panthothenyl alcohol or D-panthothenyl ethyl ether, and placenta extract;
the water soluble polymer is at least one component selected from the group consisting of: alginic acid derivative, CMC (carboxymethyl cellulose) derivative, Tamarindus indica seed polysaccharide, chitosan, poly(vinyl alcohol) and polyvinylpyrrolidone; and
the monosaccharide, disaccharide or oligosaccharide is at least one component selected from the group consisting of: trehalose and cyclodextrin.

6. The manufacture of an agent as claimed in any one of claims 1 to 5 for use in activating hair matrix cells.

7. The manufacture of an agent comprising a formulation A and a formulation B as defined in any one of claims 1 to 5 for use in activating hair matrix cells wherein the formulation A is applied to hair matrix cells; and the formulation B is applied to the same hair matrix cells after 1 to 36 hours counted from the application of the formulation A.

8. The manufacture of an agent comprising a formulation A and a formulation B as defined in any one of claims 1 to 5 for use in activating hair matrix cells wherein the formulation B is applied to hair matrix cells; and the formulation A is applied to the same hair matrix cells after 1 to 36 hours counted from the application of the formulation B.

9. Cosmetic use of an agent as claimed in any one of claims 1 to 5 in the activation of hair matrix cells.

10. Non-therapeutic use of an agent as defined in any one of claims 1 to 5 in the activation of hair matrix cells.

11. *In vitro* use of an agent as claimed in any one of claims 1 to 5 in the activation of hair matrix cells.

12. A formulation B which comprises, as main effective components, a water-soluble polymer, and at least one component selected from the group consisting of monosaccharides, disaccharides and oligosaccharides; and
is characterized in the efficiency in the promotion of hair production or hair growth by the formulation B and a formulation A comprising, at least a blood circulation promoter, and a cell activator, being alternatively applied to hair matrix cells.

13. A formulation B which comprises, as main effective components, a water-soluble polymer, and at least one component selected from the group consisting of monosaccharides, disaccharides and oligosaccharides; and
is characterized by converting the resting phase into the growth phase in the hair growth cycle.

14. A formulation A which comprises, at least a blood circulation promoter, and a cell activator; and is characterized in the efficiency in the promotion of hair production or hair growth by the formulation A and a formulation B comprising, as main effective components, a water-soluble polymer, and at least one component selected from the group consisting of monosaccharides, disaccharides and oligosaccharides, being alternately applied to hair matrix cells.
